# NEUE EUROPÄISCHE PATENTSCHRIFT

(11) **EP 0 613 688 B2**
(45) Veröffentlichungstag und Bekanntmachung des Hinweises auf die Entscheidung über den Einspruch: **18.08.2004**
(45) Hinweis auf die Patenterteilung: 27.09.2000
(21) Anmeldenummer: 93110176.0
(22) Anmeldetag: 25.06.1993
(51) Int. Cl.: A61K 33/14, A61M 1/16

(54) **Verfahren zur Bereitung von bicarbonathaltigen Dialysierflüssigkeiten für die Hämodialyse**
Process for preparing a bicarbonate containing dialysis liquid for the hemodialysis
Procédé pour la préparation de liquides de dialyse contenant du bicarbonate pour l'hémodialyse

(30) Priorität: 19.02.1993 DE 4305101
(43) Veröffentlichungstag der Anmeldung: 07.09.1994
(73) Patentinhaber: Schäl, Wilfried, Dr.-Ing., 61350 Bad Homburg (DE)
(72) Erfinder: Schäl, Wilfried, Dr.-Ing., 61350 Bad Homburg (DE)
(74) Vertreter: Luderschmidt, Schüler & Partner GbR

(56) Entgegenhaltungen:
- EP-A- 0 022 922
- EP-A- 0 086 553
- EP-A- 0 278 100
- EP-A- 0 399 549
- EP-A- 0 399 918
- EP-A- 0 490 307
- P. Keshaviah et al., Artificial Organs, 10(3), pp. 189-194, Raven Press, New York (1986)
- "Grundlagen der Dialysetechnik", Herausgeber CONZEMA GmbH - Kuratorium für Heimdialyse e.V., 10/97 (ISBN 3-8177-9270-0)
- Standardzulassungen für Fertigarzneimittel, Mai 2000, Band 1, Seite 1, Deutscher Apotherkerverlag Stuttgart, Govi-Verlag GmbH Frankfurt

## Beschreibung

### Anwendungsgebiet

Die Erfindung bezieht sich auf ein Verfahren zur Bereitung von bicarbonathaltigen Dialysierflüssigkeiten für die Hämodialyse. Für eine Dialysebehandlung werden etwa 120 - 180 Liter Dialysierflüssigkeit benötigt, bei der es sich um eine wäßrige Lösung von folgender typischer Zusammensetzung handelt:

| Komponente | Konzentration (mmol/Liter) |
|---|---|
| Na⁺ | 139 |
| K⁺ | 2,5 |
| Ca⁺⁺ | 1,65 |
| Mg⁺⁺ | 0,6 |
| Cl⁻ | 111 |
| HCO₃ | 32 |
| Acetat⁻ | 3 |

Die Probleme einer geeigneten, den physiologischen Erfordernissen angepaßten Zusammensetzung der Dialysierflüssigkeit sind z.B. in dem Buch "Replacement of Renal Function by Dialysis" (Herausgeber: W. Drukker, F.M. Parsons, J.F. Maher; Martinus Nijhoff Medical Division, Den Haag 1978) im Hauptabschnitt "The Composition of Dialysis Fluid" (Autor: F.M. Parsons, A.M. Davison) dargelegt. Zahlreiche neuere Angaben hierzu finden sich in dem Buch "Blutreinigungsverfahren" (Herausgeber: H.E. Franz, 4. Auflage; Georg Thieme Verlag; Stuttgart, New York, 1990) und der dort zitierten Literatur.

Es ist bekannt und wird auch aus diesen Veröffentlichungen deutlich, daß eine Anpassung der Zusammensetzung der Dialysierflüssigkeit an den individuellen Krankheitszustand des einzelnen Patienten zu den wesentlichen Erfordernissen für eine Optimierung der Hämodialysebehandlung zählt.

Besondere Aufmerksamkeit erfordert diese Frage bei Dialysepatienten mit akuten oder chronischen Nebenerkrankungen, bei Kindern, alten und geschwächten Patienten, ferner bei akutem Nierenversagen. In solchen Fällen muß aufgrund aktueller diagnostischer Ergebnisse und unter Berücksichtigung der gleichzeitig angewandten medikamentösen Therapie die Rezeptur der Dialysierflüssigkeit häufig korrigiert werden. Aber auch beim "normalen" Patienten ist eine sorgfältige Anpassung der Zusammensetzung sehr wichtig, um die Entwicklung von Langzeitschaden zu vermeiden.

Die technischen Mittel, die für eine individuelle Anpassung der Dialysierflüssigkeit zur Verfügung stehen, sind aber bisher unzureichend. Es ist für diese Zwecke noch kein System bekannt, das einfach zu handhaben wäre und dieses Problem mit geringen Kosten und einer einfachen gerätetechnischen Ausrüstung lösen würde.

Die Verträglichkeit der Dialyse für den Patienten kann verbessert werden, indem die Konzentration bestimmter Inhaltsstoffe im Verlauf der einzelnen Behandlung stufenweise oder stetig verändert wird. Diese Methode wird als "Profilierung" der Dialysierflüssigkeit bezeichnet. Als vorteilhaft zur Vermeidung unerwünschter Nebenwirkungen hat sich insbesondere erwiesen, in der Anfangsphase eine hohe Natriumkonzentration (z.B. 150 mmol/l) anzuwenden, in der Schlußphase eine niedrige Natriumkonzentration (z.B. 130 mmol/l). Bei Patienten mit einer überhöhten Kaliumkonzentration, besonders in Fällen akuten Nierenversagens, kann eine zu rasche Absenkung des Serum-Kaliumspiegels gefährlich sein. Daher wird empfohlen, in solchen Fällen die Kaliumkonzentration in der Dialysierflüssigkeit im Verlauf der Behandlung stufenweise von relativ hohen auf niedrige Werte zu senken, bis hin zu einer kaliumfreien Dialysierflüssigkeit.

Auch solche medizinisch dringend wünschenswerten Maßnahmen lassen sich mit den verfügbaren Mitteln bisher nur unbefriedigend verwirklichen.

### Stand der Technik

Mit Rücksicht auf unterschiedliche physiologische Erfordernisse wurden schon bisher unterschiedlich zusammengesetzte Dialysierflüssigkeiten eingesetzt, die sich vor allem durch ihren Gehalt an Natrium-, Kalium-, Calcium- und Magnesiumsalzen und Glucose unterscheiden. Dieser kann z.B. in folgenden Bereichen variieren (Angaben in mmol/Liter):

| | |
|---|---|
| Na⁺ | 130 - 150 |
| K⁺ | 0 - 5 |
| Ca⁺⁺ | 0,3 - 2,5 |
| Mg⁺⁺ | 0 - 1,2 |
| Glucose | 0 - 15 |

In der medizinischen Literatur finden sich Vorschläge, auch andere, bisher wenig gebräuchliche Substanzen einzusetzen, z.B. Aminosäuren.

Wegen der großen Dialysierflüssigkeitsmenge, die für eine typische 4- bis 6-stündige Behandlung benötigt wird, ist es üblich, die Dialysierflüssigkeit am Verwendungsort durch Verdünnen handelsüblicher Konzentrate mit Wasser herzustellen. Zu diesem Zweck sind die Dialysegeräte mit einer Proportionierungseinrichtung ausgestattet, die Wasser und Konzentrat(e) in dem vorgesehenen Volumenverhältnis mischt.

Bei Dialysierflüssigkeiten der angegebenen Zusammensetzung besteht das generelle Problem, daß Calcium und Magnesium in Verbindung mit Bicarbonat zur Bildung unlöslicher Niederschläge neigen, so daß die Lösung nicht über längere Zeit stabil bleibt. Dies gilt in noch höherem Maße bei erhöhten Konzentrationen, insbesondere also bei dem Versuch, ein Konzentrat einer solchen Dialysierlösung herzustellen. Zur Lösung dieses Problems sind in der Praxis zwei Verfahren gebräuchlich:
I) Acetat-Dialyse: Die gesamte Menge des Bicarbonats wird durch Acetat ersetzt. Da Acetat mit Calcium und Magnesium bei den in Betracht kommenden Konzentrationen keine unlöslichen Ausfällungen bildet, können auf diese Weise alle Lösungskomponenten in einem einzigen Konzentrat vereinigt werden. Die handelsüblichen Konzentrate für die Acetat-Hämodialyse sind in der Regel 35-fach, d.h. sie müssen mit Wasser auf das 35-fache ihres Anfangsvolumens verdünnt werden, um die gebrauchsfertige Dialysierflüssigkeit zu erhalten. Ein Vorteil dieser Acetat-Konzentrate ist, daß sie autosteril sind.
   Der Ersatz des gesamten Bicarbonats durch Acetat ist jedoch unphysiologisch. Aus diesem Grunde gilt die Acetat-Dialyse, die in der Vergangenheit als Standardverfahren eingeführt war und noch immer nicht außer Gebrauch ist, als medizinisch nicht optimal.
II) Bicarbonat-Dialyse: Anstelle eines einzigen Konzentrats werden zwei Konzentrate eingesetzt, von denen das eine den Bicarbonat-Anteil und das andere den Calcium- und Magnesium-Anteil (jeweils neben anderen Bestandteilen) enthält. Diese beiden Konzentrate werden dem Hämodialysegerät getrennt voneinander zugeführt und getrennt proportioniert und mit Wasser verdünnt, um die Dialysierflüssigkeit zu erhalten. Die Tendenz zur Bildung unlöslicher Niederschläge besteht bei diesem Verfahren erst nach dem Mischen der Komponenten. Infolge der starken Verdünnung und bei richtiger Einstellung des pH-Werts, die durch Zugabe einer physiologisch verträglichen Säure erfolgt, ist sie jedoch zu vernachlässigen.

Die Notwendigkeit, zwei Konzentrate zu verwenden, erschwert allerdings die Handhabung beträchtlich und stellt aus technischer Sicht einen erheblichen Nachteil der Bicarbonat-Dialyse gegenüber der Acetat-Dialyse dar. Außerdem beträgt das Gesamtvolumen der flüssigen Konzentrate typischerweise mehr als das Doppelte im Vergleich zur Acetat-Dialyse. Zur Erzeugung von 170 Liter Dialysierflüssigkeit, etwa entsprechend dem typischen Höchstbedarf für eine Dialysebehandlung, werden bei der Acetat-Dialyse etwa 5 Liter Konzentrat benötigt, bei der Bicarbonat-Dialyse dagegen etwa 11-13 Liter. Ursache hierfür ist die geringe Wasserlöslichkeit des Natriumbicarbonats im Vergleich zu der des Natriumacetats. Darüber hinaus erfordert das für die beiden Teilkonzentrate notwendige doppelte Proportionierungssystem, einschließlich der dafür notwendigen Überwachungseinrichtungen, einen höheren gerätetechnischen Aufwand. Trotz dieser Erschwernisse hat die Bicarbonat-Dialyse angesichts ihrer medizinischen Vorteile die Acetat-Dialyse zunehmend verdrängt.

In der Europäischen Patentschrift 0 022 922 werden alle Möglichkeiten einer Aufteilung der Lösungsbestandteile einer Bicarbonat-Dialysierflüssigkeit auf zwei Konzentrate betrachtet, die sich aus den bereits erwähnten physikochemisch vorgegebenen Regeln ergeben:
a) Das eine Konzentrat muß die notwendige Gesamtmenge des Natriumbicarbonats enthalten.
b) Das andere Konzentrat muß die notwendige Gesamtmenge der Calcium- und ggf. Magnesiumionen und die Gesamtmenge einer (physiologisch verträglichen) Säure zur Einstellung des pH-Werts enthalten.
Alle übrigen Bestandteile, insbesondere Natrium- und Kaliumchlorid, können beliebig auf die beiden Konzentrate verteilt werden.

Das Europäische Patent EP 0 086 553 bezieht sich auf eine spezielle Aufteilung der Lösungsbestandteile, die einen Sonderfall dieser prinzipiellen Möglichkeiten darstellt:
a) Das eine Konzentrat enthält die Gesamtmengen des Natriumbicarbonats und des Natriumchlorids und ggf. des Kaliumchlorids und der Glucose.
b) Das zweite Konzentrat enthält nur die Calcium- und ggf. Magnesiumsalze und die zur Einstellung des pH-Werts notwendige Säure.
Diese Aufteilung der Lösungskomponenten hat den Vorteil, daß von dem zweiten Konzentrat nur eine sehr kleine Menge benötigt wird, die somit sehr einfach zu handhaben ist. Für eine Dialysebehandlung reichen etwa 150 ml dieses Konzentrats aus. Wenn nur der Calcium- und Magnesiumgehalt der Dialysierflüssigkeit verändert werden soll, genügt es also, diese kleine Konzentratmenge in ihrer Zusammensetzung zu variieren. Wenn auch der Natrium-, Kalium- oder Glucosegehalt verändert werden muß, was weit häufiger der Fall ist, müssen jedoch beide Konzentrate verändert werden. Da das Volumen des ersten Konzentrats typischerweise 12,85 Liter beträgt, ist dies nur mit großem Aufwand zu verwirklichen.

In EP 0 086 553 sind auch andere Rezepturen von Konzentraten aus der US-Patentschrift 4,202,760 (Storey) und aus Veröffentlichungen der Firmen Bio-Systems und Renal Systems zitiert. Bei diesen ist, wie auch bei dem in EP-A-0 022 922 angeführten ersten Beispiel, ein Teil des Natriumchlorids mit dem Natriumbicarbonat in einem ersten Konzentrat vereinigt, während das übrige Natriumchlorid sowie Calcium- und Magnesiumsalze und die zur Einstellung des pH-Werts notwendige Säure in dem zweiten Konzentrat enthalten sind. Das Volumenverhältnis der beiden Konzentrate liegt in diesen Fällen zwischen 2:1 und 1:1. Das jeweils benötigte Konzentratvolumen ist dabei so groß, daß auch nach diesem bekannten Stand der Technik eine Anpassung der Zusammensetzung an individuelle physiologische Erfordernisse auf große Handhabungsprobleme stößt.

Nach dem realen, praktischen Stand der Technik ist bei der Bicarbonat-Dialyse weit überwiegend folgende Kombination von Konzentraten in Gebrauch:
(a) eine konzentrierte (8,4-prozentige = 1-molare) Lösung von Natriumbicarbonat unter der Handelsbezeichnung "Basisches Bicarbonat-Hämodialysekonzentrat". Diese stellt, bezogen auf die übliche Brutto-Konzentration von Bicarbonat von 35 mmol/Liter in der gebrauchsfertigen Dialysierflüssigkeit, ein 28,57-faches Konzentrat dar.
(b) ein 35-faches Konzentrat der übrigen Lösungsbestandteile der Dialysierflüssigkeit, das wegen des zur Korrektur des pH-Werts enthaltenen Zusatzes von Essigsäure als "Saures Bicarbonat-Hämodialysekonzentrat" bezeichnet wird, jedoch selbst kein Bicarbonat enthält.

Bei diesen handelsüblichen Konzentraten werden zur Erzeugung von 170 Liter Dialysierflüssigkeit, entsprechend dem typischen Höchstbedarf für eine Dialysebehandlung, benötigt: 5,95 Liter des basischen Hämodialysekonzentrats und 4,86 Liter des sauren Hämodialysekonzentrats, insgesamt also 10,81 Liter.

Das saure Bicarbonat-Hämodialysekonzentrat enthält hierbei diejenigen Bestandteile (Natrium-, Kalium-, Calcium- und Magnesiumsalze und Glucose), deren physiologische Anpassung wichtig ist, so daß durch eine geeignete Wahl des sauren Konzentrats die Möglichkeit der Anpassung gegeben ist. Nachteilig bleibt dabei jedoch, daß es sich um ein relativ großes Flüssigkeitsvolumen handelt, das nicht leicht zu handhaben ist.

Eine "Profilierung" der Dialysierflüssigkeit, also eine kontinuierliche oder gestufte Konzentrationsveränderung, insbesondere der Natriumionen, wird behelfsweise derart praktiziert, daß die Dosierung des sauren Konzentrats kontinuierlich oder stufenweise verändert wird. Dies hat jedoch den Nachteil, daß sich dann die Konzentration aller Inhaltsstoffe im gleichen Verhältnis verändert. Große Veränderungen, z.B. die erwähnte Absenkung der Kaliumkozentration von 4 mmol/l auf 0 mmol/l im Verlauf einer Akutdialyse, sind auf diese Weise nicht realiserbar. Außerdem ist bei vielen Hämodialysegeräten die Proportionierung nicht veränderbar.

Zur Berücksichtigung unterschiedlicher therapeutischer Anforderungen in Abhängigkeit von unterschiedlichen Krankheitszuständen der Patienten und unterschiedlichen Behandlungsbedingungen stellt die pharmazeutische Industrie eine Vielfalt unterschiedlich zusammengesetzter Hämodialysekonzentrate her, sowohl Acetat-Hämodialysekonzentrate als auch saure Konzentrate für die Bicarbonat-Hämodialyse.

Handelsüblich werden die Konzentrate zu diesem Zweck z.B. in Kanistern von 5 - 10 Litern Inhalt geliefert. Ein Kanister mit dem Konzentrat, das nach Art und Zusammensetzung vom Arzt unter Berücksichtigung individueller Erfordernisse und therapeutischer Zielsetzungen ausgewählt wurde, wird dem betreffenden Dialysegerät beigestellt, und das Konzentrat wird von dem Gerät über einen Schlauch aus dem Kanister aufgenommen. Das zum Verdünnen der Konzentrate benötigte aufbereitete Wasser wird in Krankenhäusern im allgemeinen allen Hämodialysegeräten gemeinsam über ein zentrales Versorgungssystem zugeführt.

Der Vorteil der individuellen Anpassung wird dabei jedoch mit großem Aufwand erkauft:
a) Für das auf Dialysestationen tätige Personal bedeutet der Transport der relativ schweren Konzentratbehälter eine erhebliche Belastung.
b) Die Lagerhaltung eines großen Sortiments unterschiedlicher Konzentrate in derart großen Behältern erfordert erheblichen Aufwand. Für eine Dialysestation mit 20 Behandlungsplätzen liegt die typische Vorratsmenge für eine Woche in der Größenordnung von 200-300 Behältern.
c) Reste von nicht aufgebrauchtem Konzentrat, die nach der Behandlung im Behälter verblieben sind, sollen aus hygienischen Gründen normalerweise nicht weiter verwendet werden. Die Beseitigung dieser Reste, die durchaus bis zu 30 Prozent der eigentlichen Bedarfsmenge betragen können, bedeutet eine Verschwendung und stellt darüber hinaus eine nicht unbeträchtliche Umweltbelastung dar.
d) Die gebrauchten leeren Behälter, bei denen es sich gewöhnlich um Kanister aus hochwertigen Thermoplasten handelt, sind nach bisherigem Stand der Technik und unter Berücksichtigung wirtschaftlich/organisatorischer Kriterien für den gleichem Zweck nicht wieder verwendbar. Ihre Rückführung in den Rohstoffkreislauf durch Recycling ist ebenfalls kein sehr wirtschaftliches Verfahren.

Angesichts der genannten Handhabungs- und Lagerungsprobleme ist es heute vielfach üblich, auf Dialysestationen eine zentrale Konzentratversorgung vorzusehen. Hierbei wird parallel zum Wasserversorgungsnetz ein Konzentratversorgungsnetz mit Zapfstellen an den einzelnen Dialyseplätzen zum Anschließen der Dialysegeräte installiert. Dieses Konzentratversorgungsnetz wird aus Konzentrat-Großbehältern von z.B. 500 - 1000 Liter Inhalt gespeist, die nach Bedarf ausgewechselt werden.

Im Falle der Bicarbonat-Hämodialyse muß das Leitungssystem für die zentrale Konzentratversorgung allerdings doppelt ausgeführt sein, um das Bicarbonat-Konzentrat und das "saure" Konzentrat der übrigen Lösungsbestandteile den Dialysegeräten getrennt zuzuführen.

Bei einer zentralen Konzentratversorgung entfallen die erwähnten Transport- und Lagerhaltungsprobleme für das Konzentrat innerhalb der Dialysestation, die Großbehälter sind wiederverwendbar, und die Restmengen an Konzentrat, die evtl. verworfen werden müssen, sind vergleichsweise sehr gering.

Ein schwerwiegender Nachteil der zentralen Konzentratversorgung ist jedoch, daß eine individuelle Anpassung der Zusammensetzung an die Bedürfnisse des einzelnen Dialysepatienten dann nicht mehr möglich ist, da über ein Konzentratversorgungsnetz jeweils nur eine Konzentratsorte verteilt werden kann. Der Arzt ist in diesem Falle gezwungen, eine "mittlere" Kontratzusammensetzung zu wählen, die den Bedürfnissen eines hypothetischen "Durchschnittspatienten" entspricht. Da sehr unterschiedliche Ansichten über die richtige Zusammensetzung des Konzentrats für den "Durchschnittspatienten" bestehen, ist die pharmazeutische Industrie genötigt, auch für die zentrale Konzentratversorgung in Großbehäftern eine Vielzahl unterschiedlich zusammengesetzter Konzentrate zu produzieren und bereitzuhalten, so daß ein Teil des denkbaren Rationalisierungseffekts wieder verlorengeht.

### Aufgabenstellung

Hauptsächliches Ziel der Erfindung ist es, ein anpassungsfähigeres und wirtschaftlicheres Verfahren zur Bereitung der Dialysierflüssigkeit für die Hämodialyse unter Vermeidung der meisten der vorgenannten Nachteile zu schaffen.

Insbesondere ist die Erfindung darauf gerichtet, ein Verfahren zu schaffen, das die Bereitung und Anwendung von Dialysierflüssigkeiten, deren Zusammensetzung individuell dem physiologischen Zustand und den therapeutischen Erfordernissen des einzelnen Patienten angepaßt ist, wesentlich erleichtert.

Eine weiteres Ziel ist, dieses Verfahren mit einfachen Dialysegeräten durchführen zu können, auch unter Benutzung vorhandener Dialysegeräte herkömmlicher Bauart, ohne hierfür spezielle und kostspielige Zusatzeinrichtungen zu benötigen.

Außerdem zielt die Erfindung darauf ab, bereits vorhandene arbeitserleichternde Installationen zur zentralen Konzentratversorgung für die Dialyse benutzen zu können und trotzdem die Zusammensetzung der Dialysierflüssigkeit den individuellen Erfordernissen jedes einzelnen Patienten anpassen zu können.

Ferner sollte mit der Erfindung eine einfache und wirtschaftlich vertretbare Möglichkeit geschaffen werden, die Zusammensetzung der Dialysierflüssigkeit im Verlauf der Dialysebehandlung zu variieren (Profilierung der Dialysierflüssigkeit).

### Lösung

Diese Ziele werden im wesentlichen durch die im folgenden angegebene Kombination von Merkmalen erreicht. Zusammenfassend, auch unter Berücksichtigung weiterer Ausgestaltungen, sieht die Erfindung vor:
1) Zur Bereitung der Dialysierflüssigkeit werden verwendet: (a) ein einheitliches, standardisiertes, hauptsächlich Natriumchlorid und Natriumbicarbonat enthaltendes Grundkonzentrat und (b) ein individuell auswählbares, den physiologischen Erfordernissen des Patienten angepaßtes Zusatzkonzentrat (Individualkonzentrat), das die übrigen Lösungskomponenten enthält, darunter einen Anteil von Natriumchlorid, der den physiologischen Variationsbereich abdeckt.
2) Das Grundkonzentrat liefert mindestens etwa 80 Prozent, typischerweise etwa 90-95 Prozent des Natriumchlorids und die Gesamtmenge des Natriumbicarbonats und kann einen stabilisierenden Zusatz von Natriumacetat aufweisen. Es liefert, bezogen auf die gebrauchsfertige Dialysierflüssigkeit, Natriumionen in einer Konzentration, die an oder knapp unterhalb der unteren Grenze des in Betracht kommenden physiologischen Bereiches liegt, d.h. im Bereich 125 - 135 mmol/Liter, vorzugsweise etwa 130 mmol/Liter.
Das molare Verhältnis der vom Grundkonzentrat gelieferten Ionen liegt in folgenden Bereichen: Bicarbonat/Natrium maximal 0,32; Acetat/Natrium maximal 0,03.
3) Das bevorzugte Grundkonzentrat ist ein 15- bis 18-faches flüssiges Konzentrat.
4) Das Grundkonzentrat kann aber auch in folgenden Formen zur Verfügung gestellt werden:
(a) in Form von zwei flüssigen Einzelkonzentraten, d.h. im wesentlichen einer konzentrierten Natrium- chlorid-Lösung und einer konzentrierten Natriumbicarbonat-Lösung, die wie das einzelne Grundkonzentrat einen Zusatz von Natriumacetat aufweisen können,
(b) in Form eines portionsweise abgewogenen Gemisches der Salze, das zum Auflösen in Wasser am Ort seiner Anwendung bestimmt ist,
(c) in Form eines homogenen Salzgemisches in tablettierter/granulierter Form, das zu einer quasi-kontinuierlichen Dosierung geeignet ist,
(d) wie (c), jedoch in Form vorgefertigter Körper (z.B. Stangen), mengenmäßig evtl. angepaßt an den Bedarf für eine Dialyse.

5) Das Individualkonzentrat liefert Natrium-, Kalium-, Calcium-, und Magnesiumionen sowie Glucose gemäß folgender Tabelle, die sich auf ein 130 mmol/l Natriumionen lieferndes Grundkonzentrat bezieht (Angaben in mmol/l, bezogen auf die Konzentration in der gebrauchsfertigen Dialysierflüssigkeit):

| | mittlerer Wert | typischer Bereich | maximaler Bereich |
|---|---|---|---|
| Natriumionen | 9 | 5...14 | (0...25) |
| Kaliumionen | 2 | 1...4 | (0...5) |
| Calciumionen | 1,6 | 0,75...2 | (0,3...2,5) |
| Magnesiumionen | 0,6 | 0,4...1 | (0...1,2) |
| Glucose | 5 | 0...10 | (0...15) |

Falls das Grundkonzentrat eine niedrigere/höhere Natriumkonzentration als 130 mmol/l aufweist, ist die Differenz durch einen entsprechend höher/niedriger zu wählenden Natriumgehalt des Individualkonzentrats auszugleichen.
Das Individualkonzentrat enthält außerdem mindestens eine physiologisch verträgliche Säure, vorzugsweise Salzsäure (HCl), in einer Konzentration, bezogen auf die gebrauchsfertige Dialysierflüssigkeit, von 1,5 - 4 mmol/Liter. Diese kann ganz oder teilweise durch andere physiologisch verträgliche Säuren, insbesondere Essigsäure, ersetzt sein, jedoch so, daß die Gesamtkonzentration der Acetationen in der gebrauchsfertigen Dialysierflüssigkeit 5 mmol/l nicht überschreitet.
6) Das Individualkonzentrat ist ein 120- bis 250-faches, vorzugsweise etwa 170-faches flüssiges Konzentrat, so daß das für eine Dialysebehandlung benötigte Volumen von typischerweise etwa 1 Liter in einem gewöhnlichen Infusionsbeutel zur Verfügung gestellt werden kann.
7) Alternativ kann das Individualkonzentrat in Form eines vorgefertigten, portionsweise abgewogenen Gemisches der Substanzen zur Verfügung gestellt werden, das zum Auflösen in Wasser am Ort seiner Anwendung bestimmt ist.
8) Entsprechend dem Mengenverhältnis und der Löslichkeit der Lösungsbestandteile beträgt - bei Anwendung der Konzentrate in flüssiger Form - das Volumenverhältnis des Grundkonzentrats und des Individualkonzentrats etwa 7:1 bis 16:1, typischerweise etwa 11 : 1.
9) Ein Grundkonzentrat in Form eines Trockenkonzentrats wird chargenweise oder kontinuierlich gelöst, wobei entweder ein mehr oder weniger konzentriertes flüssiges Grundkonzentrat gebildet wird, oder es kann in Verbindung mit dem Lösungsvorgang sogleich eine Verdünnung auf die Endkonzentration entsprechend der fertigen Dialysierflüssigkeit erfolgen.
10) Das flüssige Grundkonzentrat oder durch Lösen der Trockensubstanz hergestellte Grundkonzentrate oder Zwischstufen bis hin zu einer auf die Endkonzentration verdünnten Grund-Dialysierflüssigkeit werden mehreren Dialysegeräten vorzugsweise über ein zentral gespeistes Verteilungsnetz zugeführt.
11) Zur Profilierung der Dialysierflüssigkeit, d.h. zur Veränderung der Konzentration bestimmter Inhaltsstoffe im Verlauf der Dialyse, ist die Möglichkeit vorgesehen, zwei unterschiedliche Individualkonzentrate getrennt nacheinander oder aber zusammen in zeitlich variablem Mengenverhältnis zuzuführen.

### Beschreibung der Abbildungen

Die zu der nachfolgenden Beschreibung gehörigen Abbildungen zeigen:
Fig.1 - Fig.4 Diagramme zur Beschreibung des Verhältnisses der Lösungsbestandteile im Grundkonzentrat,
Fig.5 ein Diagramm, das den pH-Wert der Dialysierflüssigkeit in Abhängigkeit vom Säurezusatz angibt,
Fig.6 das Schema einer Hämodialyseeinrichtung mit mehreren Dialysegeräten, die gemeinsam mit einem Grundkonzentrat oder einer Grund-Dialysierflüssigkeit versorgt werden mit unterschiedlichen Arten der Zuführung des Individualkonzentrats,
Fig.7 eine Anordnung in schematischer Darstellung zur Bereitung von Grundkonzentrat/Grund-Dialysierflüssigkeit aus einem granulierten Gemisch der Trockensubstanz,
Fig.8 eine Anordnung in schematischer Darstellung zur Bereitung von Grundkonzentrat/Grund-Dialysierflüssigkeit aus der Trockensubstanz in Form vorgefertigter Formkörper,

### Beschreibung von Ausführungsbeispielen

### Beispiel 1.0:

Ein flüssiges, 15-faches Grundkonzentrat wurde durch Auflösen folgender Salzmengen pro Liter Konzentrat hergestellt:

| | |
|---|---|
| Natriumchlorid | 82,85 g |
| Natriumbicarbonat | 42,85 g |
| Natriumacetat (x 3 H₂O) | 3,06 g |

Es liefert in der gebrauchsfertigen Dialysierflüssigkeit

| | |
|---|---|
| Natriumionen | 130 mmol/l |
| Chloridionen | 94,5 mmol/l |
| Bicarbonationen | 34 mmol/l |
| Acetationen | 1,5 mmol/l |

### Beispiel 1.1:

Ein hierzu passendes, typisches, mittleres Individualkonzentrat fügt der gebrauchsfertigen Dialysierflüssigkeit Lösungsbestandteile in den in der ersten Spalte angegebenen Konzentrationen hinzu, so daß insgesamt die in der zweiten Spalte angegebenen Konzentrationen in der gebrauchsfertigen Dialysierflüssigkeit resultieren (Angaben in mmol/Liter):

| | | |
|---|---|---|
| Natriumionen | 9 | 139 |
| Kaliumionen | 2 | 2 |
| Calciumionen | 1,65 | 1,65 |
| Magnesiumionen | 0,5 | 0,5 |
| Chloridionen | 17,8 | 112,3 |
| Bicarbonationen | 0 | 34*) |
| Acetationen | 0 | 1,5 |
| Glucose | 5 | 5 |

| | | |
|---|---|---|
| *) Anmerkung: Beim Zusammenführen des Bicarbonats aus dem Grundkonzentrat und der im Individualkonzentrat enthaltenen Säure wird ein Teil des Bicarbonats in Wasser und Kohlendioxid umgewandelt. Effektiv wird daher im Gleichgewichtszustand die Bicarbonatkonzentration etwas vermindert. Dies ist eine bei der Bicarbonat-Dialyse generell bekannte Erscheinung. | | |

Das angegebene typische Individualkonzentrat von mittlerer Zusammensetzung ist den normalen physiologischen Erfordernissen eines erwachsenen, gut angepaßten Dialysepatienten ohne akute oder chronische Nebenerkrankungen angemessen.

Seine Herstellung als flüssiges, 170-faches Konzentrat erfolgt durch Auflösen folgender Substanzmengen in Wasser, bezogen auf ein Konzentratvolumen von 1 Liter, das zur Bereitung 170 Liter Dialysierflüssigkeit ausreicht (Angaben in g):

| | |
|---|---|
| Natriumchlorid | 89,43 |
| Kaliumchlorid | 25,35 |
| Calciumchlorid x 6H₂O | 61,45 |
| Magnesiumchlorid x 6H₂O | 17,28 |
| Salzsäure 37% | 42,5 |
| Glucose x 1 H₂O | 168,45 |

### Beispiel 1.2:

Für ein anderes zu dem Grundkonzentrat nach Beispiel 1.1 passendes Individualkonzentrat gelten die Angaben der folgendes Tabelle. Die erste Spalte gibt den Beitrag des Individualkonzentrats an, die zweite Spalte die Konzentration in der gebrauchsfertigen Dialysierflüssigkeit (Angaben in mmol/Liter).

| | | |
|---|---|---|
| Natriumionen | 12 | 142 |
| Kaliumionen | 1 | 1 |
| Calciumionen | 1,25 | 1,25 |
| Magnesiumionen | 0,5 | 0,5 |
| Chloridionen | 22 | 116,5 |
| Bicarbonationen | 0 | 34 |
| Acetationen | 0 | 1,5 |
| Glucose | 5 | 5 |

Ein solches Individualkonzentrat kann z.B. für einen an Dialysepatienten in Betracht kommen, der an renaler Osteopathie, verbunden mit einem sekundären Hyperparathyreoidismus, leidet und medikamentös mit einem calciumhaltigen Phosphatbinder und hochdosiertem Vitamin D behandelt wird.

Die Herstellung als flüssiges 170-faches Konzentrat erfolgt durch Auflösen folgender Substanzmengen in Wasser, bezogen auf ein Konzentratvolumen von 1 Liter, das zur Bereitung von 170 Liter Dialysierflüssigkeit ausreicht (Angaben in Gramm):

| | |
|---|---|
| Natriumchlorid | 98,20 |
| Kaliumchlorid | 10,44 |
| Calciumchlorid x 6H₂O | 46,56 |
| Magnesiumchlorid x 6H₂O | 17,28 |
| Salzsäure 37% | 42,5 |
| Glucose x 1H₂O | 168,45 |

### Beispiel 1.3:

Die folgende Tabelle, analog zu den vorhergehenden Beispielen, gilt für ein drittes zu dem Grundkonzentrat nach Beispiel 1.0 passendes Individualkonzentrat:

| | | |
|---|---|---|
| Natriumionen | 5 | 135 |
| Kaliumionen | 3 | 3 |
| Calciumionen | 1,75 | 1,75 |
| Magnesiumionen | 0,7 | 0,7 |
| Chloridionen | 15,9 | 110,4 |
| Bicarbonationen | 0 | 34 |
| Acetationen | 0 | 1,5 |
| Glucose | 10 | 10 |

Ein solches Individualkonzentrat kann z.B. für einen an Diabetes mellitus und Bluthochdruck leidenden und zu Krämpfen neigenden Dialysepatienten in Betracht kommen.

Die Herstellung als flüssiges 170-faches Konzentrat erfolgt durch Auflösen folgender Substanzmengen in Wasser, bezogen auf ein Konzentratvolumen von 1 Liter, das zur Bereitung 170 Liter Dialysierflüssigkeit ausreicht (Angaben in Gramm):

| | |
|---|---|
| Natriumchlorid | 49,68 |
| Kaliumchlorid | 38,02 |
| Calciumchlorid x 6H₂O | 65,18 |
| Magnesiumchlorid x 6H₂O | 24,20 |
| Salzsäure 37% | 51,00 |
| Glucose x 1H₂O | 336,91 |

### Beispiele 1.4/1.5/1.6:

Dies sind Beispiele von 170-fachen Individualkonzentraten mit einem sehr hohen Gehalt gelöster Stoffe:
a) gelöste Substanzen in g/Liter des Individualkonzentrats:

| | Beispiel 1.4 | Beispiel 1.5 | Beispiel 1.6 |
|---|---|---|---|
| Natriumchlorid | 159 | 198,7 | 238,5 |
| Kaliumchlorid | 50,7 | 63,4 | 25,3 |
| Calciumchlorid | 74,5 | 74,5 | 61,5 |
| Magnesiumchlorid | 34,6 | 34,6 | 17,3 |
| Essigsäure | 25,5 | 25,5 | 25,5 |
| Glucose | 505,4 | 168,5 | 0 |

b) Beitrag zur Konzentration in der gebrauchsfertigen Dialysierflüssigkeit (mmol/l):

| | Beispiel 1.4 | Beispiel 1.5 | Beispiel 1.6 |
|---|---|---|---|
| Natriumionen | 16 | 20 | 24 |
| Kaliumionen | 4 | 5 | 2 |
| Calciumionen | 2 | 2 | 1,65 |
| Magnesiumionen | 1 | 1 | 0,5 |
| Chloridionen | 26 | 31 | 30,3 |
| Acetationen | 2,5 | 2,5 | 2,5 |
| Glucose | 15 | 5 | 0 |

Die Beispiele 1.4/1.5/1.6 demonstrieren, daß die Zusammensetzung von 170 Liter Dialysierflüssigkeit mit nur einem Liter Individualkonzentrat in erstaunlich weiten Grenzen variert werden kann.

Diese Konzentrate zeigen auch bei niedriger Temperatur (5 °C) keine Ausfällungen. Dies ist überraschend angesichts der Tatsache, daß z.B. das Individualkonzentrat gemäß Beispiel 1.4 (Dichte 1,29 g/ml) pro Liter der Lösung 0,824 kg Trockensubstanz enthält. Sein Flüssigkeitsanteil aus Wasser und Säure beträgt nur etwa 361 g pro kg Lösung (36,1 %).

Abgesehen von einem normalen Glucosegehalt liefert das Individualkonzentrat nach Beispiel 1.5 Kalium-, Calcium- und Magnesiumionen in sehr hohen Konzentrationen, die nur selten zur Anwendung kommen und noch seltener für alle Ionenarten gemeinsam. Trotzdem kann dieses Konzentrat noch 20 mmol Natriumionen pro Liter der gebrauchsfertigen Dialysierflüssigkeit liefern. Ausgehend von dem Grundkonzentrat nach Beispiel 1.0, welches Natriumionen in einer Konzentration von 130 mmol/l liefert, bedeutet dies, daß der Natriumgehalt der gebrauchsfertigen Dialysierflüssigkeit 150 mmol/l erreicht. Die Möglichkeit, den Natriumgehalt der Dialysierflüssigkeit im gesamten Bereich von 130 bis 150 mmol/l zu variieren, ist mehr als ausreichend, um den gesamten physiologisch wünschenswerten Variationsbereich abzudecken und reicht sogar aus, Natriumprofile in dem in der Literatur empfohlenen Bereich zu realisieren.

### Beispiel 2.0:

Ein 16-faches Grundkonzentrat kann durch Auflösen folgender Salzmengen pro Liter Lösung hergestellt werden:

| | |
|---|---|
| Natriumchlorid | 93,52 g |
| Natriumbicarbonat | 43,69 g |
| Natriumacetat (x 3 H₂O) | 5,44 g |

Es liefert in der gebrauchsfertigen Dialysierflüssigkeit

| | |
|---|---|
| Natriumionen | 135 mmol/l |
| Chloridionen | 100 mmol/l |
| Bicarbonationen | 32,5 mmol/l |
| Acetationen | 2,5 mmol/l |

### Beispiele 2.1/2.2:

Passend zu dem Grundkonzentrat nach Beispiel 2.0 sind im folgenden die Zusammensetzungen von 200-fachen Individualkonzentraten angegeben, und zwar eines typischen mittleren Konzentrats (Beispiel 2.1) und eines Konzentrats mit hohem Gehalt an Lösungsbestandteilen (Beispiel 2.2).
a) gelöste Substanzen in g/Liter des Individualkonzentrats:

| | Beispiel 2.1 | Beispiel 2.2 |
|---|---|---|
| Natriumchlorid | 46,8 | 116,9 |
| Kaliumchlorid | 29,8 | 74,6 |
| Calciumchlorid | 72,3 | 87,6 |
| Magnesiumchlorid | 20,3 | 40,7 |
| Salzsäure (37%) | 49,3 (2,5) | 29,6 (1,5) |
| Essigsäure (100%) | 0 | 11,8 (1) |
| Glucose | 198,2 | 495,5 |

b) Beitrag zur Konzentration in der gebrauchsfertigen Dialysierflüssigkeit (mmol/l):

| | Beispiel 2.1 | Beispiel 2.2 |
|---|---|---|
| Natriumionen | 4 | 10 |
| Kaliumionen | 2 | 5 |
| Calciumionen | 1,65 | 2 |
| Magnesiumionen | 0,5 | 1 |
| Chloridionen | 10,3 | 22,5 |
| Acetationen | 2,5 | 3,5 |
| Glucose | 5 | 12,5 |

Bei diesen Beispielen reichen für 170 Liter der gebrauchsfertigen Dialysierflüssigkeit sogar nur etwa 0,85 Liter des Individualkonzentrats aus. In Verbindung mit dem Grundkonzentrat nach Beispiel 2.0 wird der gesamte physiogisch wünschenswerte Variationsbereich der wesentlichen Lösungsbestandteile abgedeckt. Der Variationsmöglichkeit der Natriumkonzentration erstreckt sich auf den Bereich 135 - 145 mmol/l, was ebenfalls den Anforderungen entspricht, wenn man von extremen Variationen absieht, wie sie bei Natrium-Profilen wünschenswert sein können.

In den Beispielen 1.0 und 2.0 wurden zwei unterschiedliche Grundkonzentrate angegeben, obwohl ein wesentlicher Gedanke der Erfindung darin besteht, grundsätzlich in allen Fällen ein einheitliches, standardisiertes Grundkonzentrat verwenden zu können. Mit diesen Beispielen sollte nur gezeigt werden, daß unterschiedliche Wahlmöglichkeiten bei der Festlegung des standardisierten Grundkonzentrats bestehen.

Bei der Festlegung der Zusammensetzung des Grundkonzentrats sind folgende Forderungen zu berücksichtigen, die sich zum Teil widersprechen:
1) maximale Variationsbreite der Zusammensetzung der gebrauchsfertigen Dialysierflüssigkeit innerhalb des physiologischen Bereiches mit einer möglichst kleinen Menge des Individualkonzentrats,
2) geringes Volumen bei Einsatz als flüssiges Grundkonzentrat,
3) hohe Stabilität und gute Lagerfähigkeit,
4) geringer, physiologisch verträglicher Acetatgehalt.

Um einen ausreichenden Variationsbereich zur physiologischen Anpassung des Natriumgehalts zur Verfügung zu haben und das Volumen des Grundkonzentrats gering zu halten, darf der Natriumgehalt des Grundkonzentrats nicht zu hoch sein. Ein bevorzugter Wert der Natriumkonzentration sollte an der unteren Grenze des physiologischen Bereiches oder knapp darunter liegen, d.h., bezogen auf die gebrauchsfertige Dialysierflüssigkeit, zwischen etwa 122,5 und 135 mmol/l. Ein noch niedrigerer Natriumgehalt würde einen höheren Anteil von Natrium im Individualkonzentrat erfordern. Dies würde ein höheres Volumen des Individualkonzentrats erforderlich machen und damit einem anderen wesentlichen Aspekt der Erfindung widersprechen.

Die Forderung nach geringem Volumen sowie hoher Stabilität und Lagerfähigkeit des Grundkonzentrats (insbesondere bei tiefen Temperaturen) kann, wie Versuche ergeben haben, durch einen Zusatz von Natriumacetat zum Grundkonzentrat leichter erfüllt werden. Andererseits muß der Acetatgehalt in der Dialysierflüssigkeit aus physiologischen Gründen gering bleiben. Überraschenderweise verhindert ein Zusatz von Acetat im Grundkonzentrat die Ausfällung von Natriumbicarbonat, was vermutlich auf die Bildung von komplexen Bicarbonat-Acetat-Ionen zurückzuführen ist. Aufgrund von Löslichkeitsexperimenten mit verschiedenen Mischungen von Natriumbicarbonat, Natriumacetat und Natriumchlorid kann vermutet werden, daß in diesen komplexen Ionen Acetat- und Bicarbonationen im Verhältnis von etwa 1:3 vereinigt sind. Die beobachtete erhöhte Löslichkeit von Natriumbicarbonat in Natriumacetat enthaltenden Lösungen entspricht jedenfalls dieser Hypothese und diesem Zahlenverhältnis. Dieser Effekt scheint bisher nicht wahrgenommen worden zu sein und wurde jedenfalls bisher nicht dazu genutzt, das Flüssigkeitsvolumen eines Dialysekonzentrats möglichst gering zu halten.

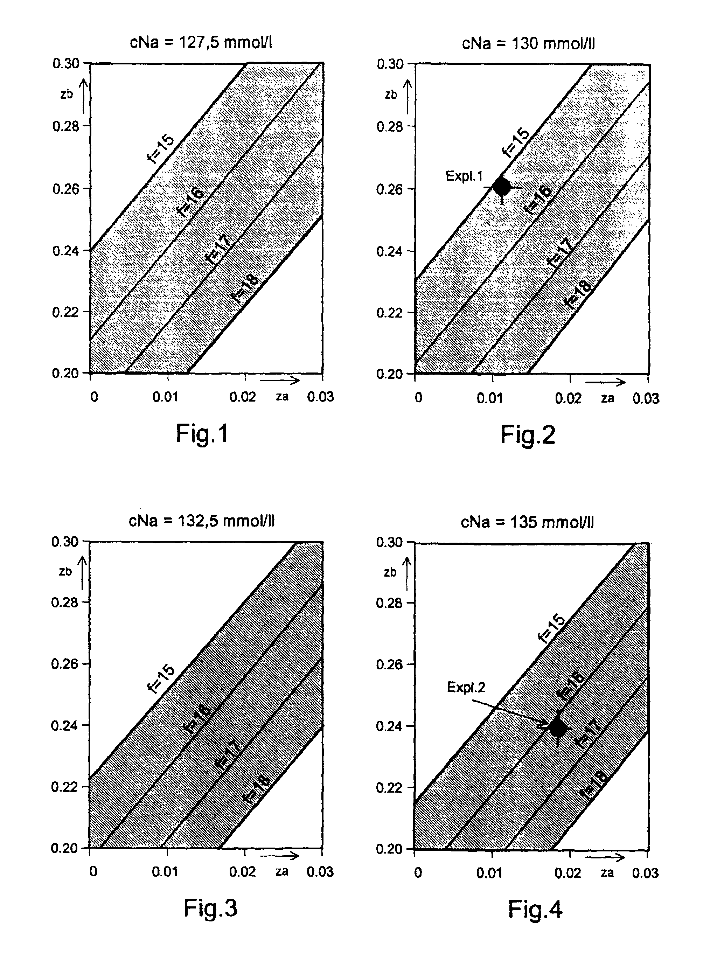

Die Diagramme Fig.1 - Fig.4 zeigen für Grundkonzentrate mit unterschiedlichem Gesamtgehalt an Natriumionen (bezogen auf die gebrauchsfertige Dialysierflüssigkeit), welche Menge Bicarbonat gelöst werden kann, wenn unterschiedliche Mengen Acetat verwendet werden. Dabei wurde von einer niedrigsten Lagerungstemperatur von 10 °C ausgegangen. Dies erscheint ausreichend angesichts der Tatsache, daß die sonst verwendete 1-molare Natriumbicarbonatlösung bereits bei Unterschreitung von 14 °C nicht mehr stabil ist. Die Größen za und zb sind wie folgt definiert: za = molares Verhältnis Acetat/Gesamtnatrium; zb = molares Verhältnis Bicarbonat/Gesamtnatrium. Der Parameter f ist der Konzentrationsfaktor, identisch mit dem volumetrischen Verdünnungsfaktor, der anzuwenden ist, um aus dem Konzentrat die Dialysierflüssigkeit zu erzeugen. Die Kurven geben die Löslichkeitsgrenze an. Ein Gemisch von Natriumchlorid, Natriumacetat und Natriumbicarbonat ist löslich, wenn der zugehörige Punkt unter der betreffenden Grenzkurve des Konzentrationsfaktors f liegt. Ohne die erwähnte Wirkung von Acetat würden die Kurven, ausgehend von ihrem Schnittpunkt mit der zb-Achse, horizontal verlaufen. Die Beispiele 1.0 und 2.0 sind in Fig.2 bzw. Fig.4 eingetragen.

Diese Untersuchungen im Zusammenhang mit den vorausgegangenen Überlegungen führen zu folgender Festlegung der Eigenschaften des Grundkonzentrats (ohne Berücksichtigung der zusätzlichen Substanzen, die von dem Individualkonzentrat geliefert werden):
a) Konzentration der Natrium-Ionen, bezogen auf die gebrauchsfertige Dialysierflüssigkeit (unter Berücksichtigung des Verdünnungsfaktors): 125 - 135 mmol/l,
b) molares Verhältnis Acetat/Natrium: 0 - 0,03,
c) Verdünnungsfaktor: 15 - 18,
d) molares Verhältnis Bicarbonat/Natrium: 0,2 - 0,32.

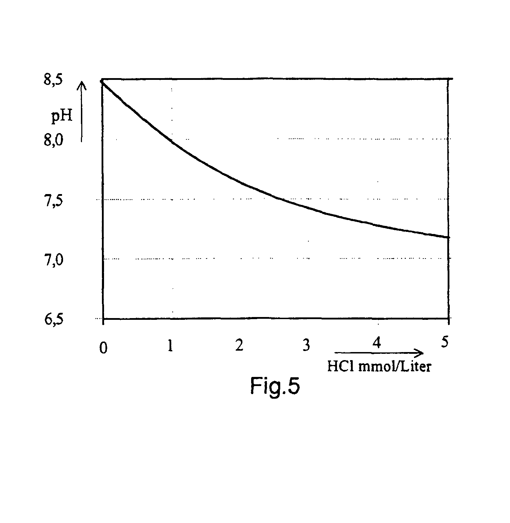

Zur Absenkung des pH-Werts der Dialysierflüssigkeit enthalten die Individualkonzentrate eine physiologisch verträgliche Säure. Hierzu wird erfindungsgemäß vorzugsweise Salzsäure verwendet, um unter Berücksichtigung eines Acetatzusatzes im Grundkonzentrat die Gesamt-Acetatkonzentration der Dialysierflüssigkeit auf maximal etwa 5 mmol/l zu begrenzen. Die Salzsäure kann jedoch unter Einhaltung dieses Grenzwertes ganz oder teilweise durch Essigsäure und/oder andere physiologisch verträgliche Säuren ersetzt werden. Der pH-Wert einer Dialysierflüssigkeit mittlerer Zusammensetzung in Abhängigkeit von dem Säurezusatz in mmol/l, bezogen auf die gebrauchsfertige Dialysierflüssigkeit, ist in dem Diagramm Fig.5 dargestellt. Die dargestellte Kurve für Salzsäure gilt im Rahmen der Meßgenauigkeit gleichermaßen auch für Essigsäure. Der normale Bereich für den Säuresatz liegt zwischen etwa 2 mmol/l und 4 mmol/l. Auch bei der Wahl des Säurezusatzes kommt eine individuelle Anpassung an die physiologischen Erfordernisse des einzelnen Patienten in Betracht.

Die für eine Dialysebehandlung benötigte Gesamtmenge des Individualkonzentrats ist aufgrund der erfindungsgemäß vorgesehenen Maßnahmen sehr gering, typischerweise etwa 1 Liter oder sogar weniger. Keines der bisher bekannten Verfahren erlaubt, mit einer derart geringen Konzentratmenge den Natrium-, Kalium-, Calcium-, Magnesium- und Glucosegehalt der Dialysierflüssigkeit über den gesamten physiologischen Variationsbereich zu verändern. Das geringe Volumen des Individualkonzentrats bietet u.a. den Vorteil, daß auch einfachere Hämodialysegeräte, insbesondere auch solche, die bisher nur für die Acetat-Dialyse ausgestattet sind, für das neue Verfahren benutzt werden können, indem zur Dosierung des Individualkonzentrats eine der üblichen volumetrischen Infusionspumpen, wie sie in Krankenhäusern gebräuchlich sind, benutzt wird. Wegen des geringen Volumens kann das Individualkonzentrat z.B. direkt in die zum Dialysator führende Leitung eingeführt werden, ohne daß eine nennenswerte Temperaturabsenkung eintritt. Bei einer Temperatur der Dialysierflüssigkeit von 37°C und einer Temperatur des Individualkonzentrats von 20°C sinkt die Temperatur um nur etwa 0,1 Grad ab.

Das Grundkonzentrat von einheitlicher Zusammensetzung wird den Dialysegeräten auf Dialysestationen in einem Krankenhaus sinnvollerweise über ein zentrales Konzentratversorgungssystem zugeführt. Das Individualkonzentrat kann dagegen angesichts seiner nur geringen Menge ohne weiteres von der Behandlungsperson zum Dialysegerät transportiert werden, wie auch andere Materialien, die für die Durchführung der Behandlung benötigt werden.

Mit diesem Vorgehen gemäß der Erfindung sind erhebliche Vorteile verbunden: Es besteht weitgehende Freiheit in der individuellen Anpassung der Zusammensetzung der Dialysierflüssigkeit an die Erfordernisse des einzelnen Patienten, da der oben angegebene Variationsbereich weitgehend alle entsprechenden Bedürfnisse abdeckt und bei Bedarf auch noch erweitert werden kann. Der Aufwand für Handhabung, Transport und Lagerhaltung ist im Vergleich zu der einleitend beschriebenen Handhabung der herkömmlichen Kanister (von z.B. 10 Litern) relativ gering. Entsprechend der generell kleineren Menge sind auch die eventuell zu vernichtenden Restmengen des Individualkonzentrats erheblich geringer, und auch das Recycling der wesentlich kleineren Behälter in Form von Flaschen oder Beuteln, wie sie in der Infusionstechnik gebräuchlich sind, ist weniger problematisch.

Ein weiterer wichtiger Vorteil ergibt sich aus der Verwendung des einheitlichen Grundkonzentrats für den gesamten Variationsbereich unterschiedlich zusammengesetzter Dialysierflüssigkeiten. Produktion, Disposition, Transport und Lagerhaltung können erheblich vereinfacht werden, was auch zu entsprechenden Kostensenkungen führen sollte.

Weitere Vorteile sind:
a) Bei einer Verteilung des Grundkonzentrats auf mehrere Behandlungsplatze über ein Leitungssystem ist für das Grundkonzentrat nur ein einfaches Leitungssystem notwendig, während für die Bicarbonat-Dialyse nach bisherigem Verfahren ein doppeltes Leitungssystem notwendig ist.
b) Unter der Voraussetzung, daß die geringe Menge des Individualkonzentrats kontinuierlich über eine Infusionspumpe oder eine ähnliche Zusatzeinrichtung zugeführt wird, muß die Proportionierungseinrichtung des Hämodialysegeräts nur eine Dosiervorrichtung für das Grundkonzentrat aufweisen, während für die Bicarbonat-Hämodialyse nach traditionellem Verfahren ein komplizierteres doppeltes Proportionierungssystem notwendig ist.
c) Das Grundionzentrat gemäß dem Verfahren der Erfindung bietet wegen seiner hohen Kochsalzkonzentration und seiner höheren Osmolarität von typischerweise etwa 4000 mosmol/l geringere Möglichkeiten für eine Vermehrung von Mikroorganismen als das bisher für die Bicarbonat-Dialyse eingesetzte reine Bicarbonatkonzentrat. Die erheblichen hygienischen Probleme, die sonst mit der Bicarbonat-Dialyse verbunden sind, vermindern sich hierdurch.

Der unter dem vorstehenden Punkt b) genannte Vorteil bedeutet in der Praxis, daß Hämodialysegeräte, die bisher nur für die Acetat-Dialyse ausgestattet und demgemäß nur mit einer Proportionierungeinrichtung für ein Konzentrat ausgerüstet sind, nun mit relativ geringfügigen apparativen Ergänzungen auch für die Bicarbonat-Dialyse benutzt werden können, noch dazu mit dem generellen Vorteil der Möglichkeit, die Zusammensetzung der Dialysierflüssigkeit individuell an die Bedürfnisse der Patienten anzupassen.

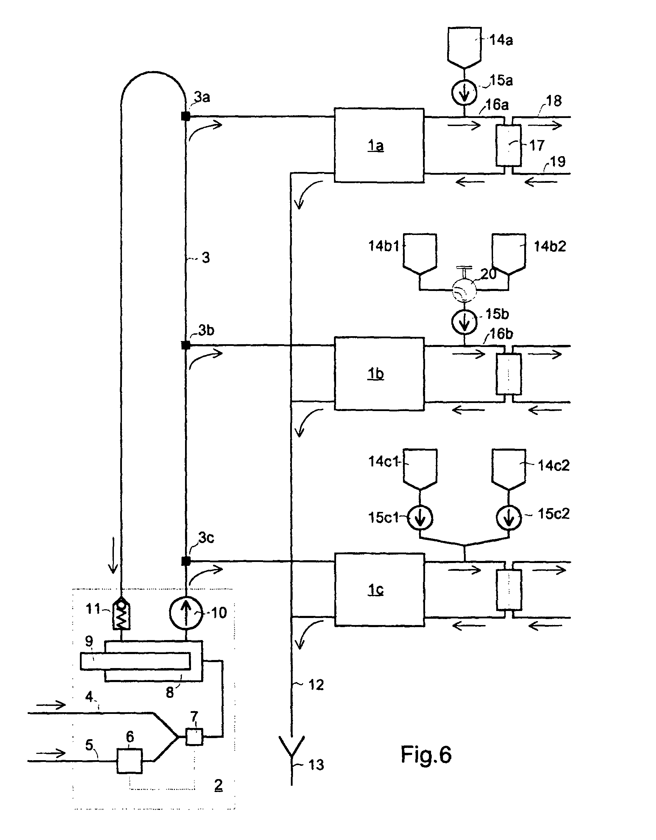

Bei der in Fig.6 gezeigten Anordnung dient eine zentrale Versorgungseinheit 2 dazu, über eine Ringleitung 3 mehreren Dialysegeräten 1, 1b, 1c ein Grundkonzentrat der in den vorherigen Beispielen beschriebenen Zusammensetzung oder eine entsprechende Grund-Dialysierflüssigkeft zuzuführen. Das Schema zeigt nur drei Dialysegeräte. Tatsächlich ist ein solches System aber zur Versorgung einer wesentlich größeren Anzahl von Dialysegeräten geeignet, wie es dem Bedarf bei Dialysestationen in Krankenhäusern entspricht.

Der schematisch dargestellte innere Aufbau der Versorgungseinheit 2 berücksichtigt den Fall, daß aus dem flüssigen Grundkonzentrat, das aus einem Vorratsgefäß über die Leitung 5 zufließt, und aufbereitetem reinem Wasser, das z.B. von einem Omkehrosmosesystem über die Leitung 4 zugeführt wird, eine Grund-Dialysierflüssigkeit gebildet wird, die bereits auf die endgültige Konzentration verdünnt ist. Bei Anwendung eines 15-fachen Grundkonzentrats nach Beispiel 1.0 werden in der Versorgungseinheit dementsprechend pro Volumeneinheit Konzentrat ca. 14 Votumeneinheiten Wasser zugesetzt, um ca. 15 Volumeneinheiten Grund-Dialysierflüssigkeit zu bilden. Das 170-fache Individualkonzentrat wird dieser Grund-Dialysierflüssigkeit an den einzelnen Dialysegeräten später hinzugefügt. Genau genommen muß daher der Wasseranteil statt 14 Volumeneinheiten 15-1-15/170 = 13,91 Volumeneinheiten betragen.

Die Proportionierung von Wasser und flüssigem Grundkonzentrat in dem genannten Verhältnis kann mit verschiedenen bekannten technischen Mitteln erfolgen, z.B. mit Dosierpumpen, die nach einem volumetrischen Prinzip arbeiten oder nach dem Prinzip des geschlossenen Regelkreises aufgrund einer Analyse des gebildeten Gemisches. Dieser letztere Fall ist in Fig.6 schematisch angedeutet. Die Konzentration der durch Mischen von Grundkonzentrat und Wasser gebildeten Grund-Dialysierflüssigkeit wird durch die Analysevorrichtung 7 gemessen und mit einem Standardwert verglichen und die Tätigkeit der Konzentrat-Dosiervorrichtung 6 so geregelt, daß die Abweichung zwischen dem gemessenen Wert (Istwert) und dem Standardwert (Sollwert) ausreichend klein ist.

Die so gebildete Grund-Dialysierflüssigkeit wird einem Rezirkulationskreislauf zugeführt, der aus einem Gefäß 8, der Pumpe 10, der Ringleitung 3 und einem Druckhalteventil 11 besteht. Die Pumpe 10 fördert die Grund-Dialysierflüssigkeit in die Ringleitung 3 mit den Anschlußstellen 3a,b,c... für die einzelnen Hämodialysegeräte. Die überschüssig zurückfließende Flüssigkeit gelangt über ein Druckhalteventil 11 in das Gefäß 8 zurück. Das Druckhalteventil 11 erhält in der Ringleitung einen bestimmten Druck aufrecht, wie er für die Versorgung der Dialsegeräte erforderlich ist.

Da die Proportionierung von Grundkonzentrat und Wasser in der zentralen Versorgungseinheit erfolgt, muß nicht jedes einzelne Hämodialysegerät mit einer Proportionierungseinrichtung ausgestattet sein. Hierdurch können wesentliche Vereinfachungen und Kosteneinsparungen erreicht werden. Außerdem entfällt das sonst notwendige Leitungsnetz zur Versorgung der Dialysegeräte mit aufbereitetem Wasser. Auch die Erwärmung der Dialysierflüssigkeit auf die Körperatur kann in der zentralen Versorgungseinheit erfolgen, indem das Gefäß 8 mit einer thermostatisch geregelten Heizvorrichtung 9 ausgestattet wird. Dadurch können die Heizvorrichtungen in den einzelnen Dialysegeräten entfallen oder durch wesentlich kleinere Heizvorrichtungen ersetzt werden, die nur der Temperaturkorrektur dienen. Eine solche zentrale Heizvorrichtung kann auch vorteilhaft dazu dienen, die gesamte Ringleitung vor und nach dem Gebrauch mit heißem Wasser zu sterilisieren, eventuell einschließlich der Flüssigkeitssysteme aller daran angeschlossenen Dialysegeräte.

Eine Anordnung entsprechend dem Schema Fig.6 ist auch geeignet, lediglich als zentrales Versorgungssystem für das Grundkonzentrat zu dienen. Das Grundkonzentrat wird dann direkt in das Gefäß 8 der Versorgungseinheit 2 geleitet. Die Einrichtungen zur Proportionierung entfallen. Das aufbereitete Wasser wird den Dialysegeräten über eine separate Ringleitung zugeführt, und jedes einzelne Dialysegerät muß mit einer eigenen Proportionierungseinrichtung ausgestattet sein.

In Fig.6 sind verschiedene Arten der Zuführung des Individualkonzentrats schematisch dargestellt. Bei dem Dialysegerät 1a wird das Individualkonzentrat aus dem Gefäß 14a, vorzugsweise einem Infusionsbeutel mit einem Volumen von etwa 1 Liter, über die volumetrische Pumpe 15a in die Leitung 16a gefördert, durch die dem Dialysator 17 die frische Dialysierflüssigkeit zufließt. Durch die Zugabe des Individualkonzentrats erhält die Dialysierflüssigkeit ihre endgültige, individuell auf den Patienten abgestimmte Zusammensetzung. Die Anordnung des Dialysators 17 mit seinen Anschlußleitungen 18 und 19 für den Blutkreislauf des Patienten entspricht im übrigen den üblichen Verhältnissen.

Bei dem Dialysegerät 1 b ist eine Anordnung zur "Profilierung" der Dialysierflüssigkeit vorgesehen. Ein Profil, d.h eine zeitlich variable Zusammensetzung der Dialysierflüssigkeit, wird durch Verwendung von zwei verschiedenen Individualkonzentraten in den Infusionsbeuteln 14b1 und 14b2 erreicht, die über eine Reguliervorrichtung 20 und die volumetrische Pumpe 15b in die zum Dialysator führende Leitung 16b gefördert werden. Die Reguliervorrichtung 20, die ein unterschiedliches Verhältnis der Strömungswiderstände in den beiden Strömungswegen einzustellen gestattet, bestimmt, in welchem Verhältnis die beiden Indiviualkonzentrate zugeführt werden. Im einfachsten Falle ist die Reguliervorrichtung 20 ein Dreiwegehahn, mit dem wahlweise zwischen dem ersten und dem zweiten Individualkonzentrat umgeschatet werden kann, so daß sich ein gestuftes Profil ergibt.

Eine weitere Möglichkeit, die Zusammensetzung der Dialysierflüssigkeit im Sinne eines kontinuierlichen Profils zu variieren, wird bei dem Dialysegerät 1c angewandt. Zwei entsprechend ausgewählte Individualkonzentrate werden aus den Infusionsbeuteln 14c1 und 14c2 über zwei volumetrische Pumpen 15c1 und 15c2 zugeführt, deren Fördergeschwindigkeiten variiert werden, und zwar im allgemeinen so, daß die Summe der pro Zeiteinheit von beiden Pumpen geförderten Volumina konstant ist.

Diese letztere Bedingung muß jedoch nicht unbedingt eingehalten werden, sondern es kann z.B. die Pumpe 15c1 mit konstanter Fördergeschwindigkeit betrieben werden, während die Fördergeschwindigkeit der Pumpe 15c2, ausgehend von dem gleichen Anfangswert, zeitlich linear abnimmt.

Dies zeigt das folgende Beispiel: Es sei beabsichtigt, unter Verwendung eines Grundkonzentrats nach Beispiel 1.0 die Zusammensetzung der Dialysierflüssigkeit im Verlauf einer Dialysebehandlung wie folgt zu variieren (Angaben in mmol/l):

| | |
|---|---|
| Natriumionen | 145 absinkend auf 135 |
| Kaliumionen | 4 absinkend auf 1 |
| Calciumionen | 1,65 konstant |
| Magnesiumionen | 0,5 konstant |
| Glucose | 10 absinkend auf 5 |

Dies kann mit folgenden Individualkonzentraten erreicht werden (Angaben in mmol/l, bezogen auf die gebrauchsfertige dialysierflüssigkeit)

| | Konzentrat 1 | Konzentrat 2 |
|---|---|---|
| Natriumionen | 5 | 5 |
| Kaliumionen | 1 | 3 |
| Calciumionen | 1,65 | 0 |
| Magnesiumionen | 0,5 | 0 |
| Chloridionen | 12,8 | 8 |
| Glucose | 5 | 5 |

Das Konzentrat 1 ist ein "normales" Individualkonzentrat in dem Sinne, daß es u.a. die zur Einstellung des pH-Werts notwendige Säure (in diesem Falle 2,5 mmol HCl pro Liter der gebrauchsfertigen Dialysierflüssigkeit) liefert. Das Konzentrat 2 ist dagegen ein Zusatzkonzentrat, das nur der Profileinstellung dient, keinen Säurezusatz aufweist und demensprechend auch nicht als selbständiges Individualkonzentrat verwendet werden könnte.

Das Konzentrat 1 wird während der gesamten Dialysedauer über die Pumpe 15c1 mit konstanter Geschwindigkeit zugeführt, während die Fördergeschwindigkeit der Pumpe 15c2, über die das Konzentrat 2 zugeführt wird, ausgehend von dem gleichen Wert im Verlauf der Dialyse linear auf Null abnimmt. Dann ergibt sich, unter Berücksichtigung aller Lösungsbestandteile, der folgende zeitliche Verlauf der Konzentrationen in der Dialysierflüssigkeit:

| | |
|---|---|
| Natriumionen | 145 absinkend auf 135 |
| Kaliumionen | 4 absinkend auf 1 |
| Calciumionen | 1,65 |
| Magnesiumionen | 0,5 |
| Chloridionen | 115,3 absinkend auf 107,3 |
| Bicarbonationen | 34 |
| Acetationen | 1,5 |
| Glucose | 10 absinkend auf 5 |

Durch die Zufuhr des Konzentrats 2 tritt - genau genommen - eine zusätzliche Volumenvergrößerung der Dialysierflüssigkeit ein. Wegen der erfindungsgemäß vorgesehenen hohen Konzentration der Indiviualkonzentrate (typischerweise 170-fach) kann dieser Effekt jedoch vernachlässigt werden, denn maximal werden die Konzentrationen der im Grundkonzentrat und im Konzentrat1 enthaltenen Lösungsbestandteile nur etwa um den Faktor 0,994 verändert.

Ein weiterer Aspekt der Erfindung liegt in der Möglichkeit, das Grundkonzentrat am Ort seiner Anwendung, z.B. in einem Krankenhaus, durch Auflösen seiner Trockensubstanz in Wasser herzustellen. Im einfachsten Falle geschieht dies dadurch, daß die Trockensubstanz in Form eines abgewogenen Salzgemisches zusammen mit der entsprechenden Wassermenge in einen Behälter gebracht und gelöst wird. Diese Lösung kann einem zentralen Versorgungssystem von der in Fig.6 gezeigten zugeführt werden.

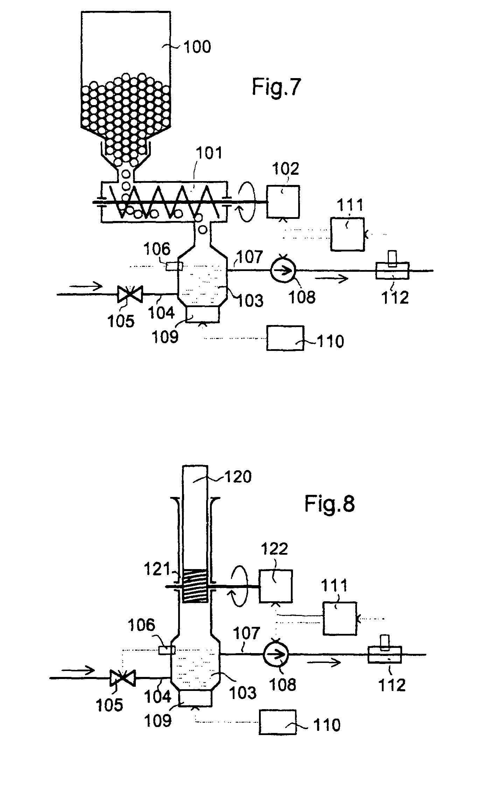

Ein anderes Ausführungsbeispiel der Erfindung, bei dem das Grundkonzentrat als Trockensubstanz angewandt wird, ist in Fig.7 angegeben. Das Grundkonzentrat wird in diesem Falle in Form einer homogenen Mischung der betreffenden Salze in gekörnter oder tablettierter Form verwendet. Dieses Trockenkonzentrat wird aus einem Behälter 100 über eine mechanische Transport- und Dosiervorrichtung 101, z.B. mit einer von dem Motor 102 angetriebenen Schnecke 101, in die Kammer 103 gefördert, wo es in Wasser aufgelöst wird. Das Wasser wird bei offenem Ventil 105 über die Leitung 104 zugeführt. Das Ventil 105 wird über den Niveausensor 106 geregelt, um den Füllstand in der Kammer 103 annähernd konstant zu halten. Die gebildete Lösung, die ein flüssiges Grundkonzentrat darstellt, wird von der Pumpe 108 über die Leitung 107 aus der Kammer abgeführt und kann z.B. dazu dienen, das Leitungsnetz eines zentralen Konzentratversorgungssystems in einem Krankenhaus zu speisen, so daß mehrere Dialysegeräte mit dem Grundkonzentrat versorgt werden. Die Anordnung nach Fig.4 könnte aber auch Bestandteil eines einzelnen Hämodialysegerätes sein und dabei die herkömmliche Art von Proportionierungseinrichtungen ersetzen. Zur Erzeugung einer Lösung von konstanter Konzentration ist z.B. eine automatische Leitfähigkeitsregelung geeignet. Ein Regler 111 mißt die aktuelle Leitfähigeit der Lösung mittels des Sensors 112 und koordiniert die Geschwindigkeiten der Pumpe 108 und der Fördereinrichtung 101,102, um die Leitfähigkeit in einem vorgegebenen Bereich zu halten.

Angesichts der unterschiedlichen Lösungsgeschwindigkeiten der in den Körnern oder Tabletten enthaltenen Substanzen hat es sich zur Erzielung einer homogenen Lösung mit konstantem Verhältnis ihrer Lösungskomponenten als zweckmäßig erwiesen, folgende Punkte zu beachten: (a) Der Gesamtgehalt an gelöster Substanz in der Kammer 103 sollte beträchtlich größer sein als die durch eine Dosiereinheit des Trockenkonzentrats (Korn, Tablette) jeweils hinzugefügte Festsubstanzmenge.
(b) Die Auflösungsgeschwindigkeit sollte hoch genug sein, um die Ansammlung ungelöster Substanz in der Kammer 103 zu vermeiden. Dies kann durch Wahl eines angemessenen Kammervolumens und durch Erhöhung der Auflösungsgeschwindigkeit mit einer Rührvorrichtung oder mittels Ultraschall, erreicht werden. Für den letzteren Zweck ist am Boden der Kammer ein Ultraschallwandler 109 angeordnet, der von einem Hochfrequenz-Leistungsgenerator 110 gespeist wird.

Eine andere, ähnlich vorteilhafte Ausgestaltung der Erfindung besteht in der Anwendung eines festen Grundkonzentrats in Form vergefertigter stabförmiger oder ähnlich geformter Stücke, z.B. in einer Größe, die dem Bedarf für eine Dialysebehandlung entspricht, d.h. etwa 1 kg. Solches Material kann durch Pressen oder Extrudieren einer homogenen Mischung hauptsächlich von Natriumchlorid und Natriumbicarbonat hergestellt werden, nach Bedarf unter Verwendung eines physiologisch verträglichen Bindemittels (z.B. Gelatine, Polyvinylpyrrolidon oder Polysaccharide). Entsprechend dieser Ausgestaltung werden die vorgefertigten Stücke des festen Grundkonzentrats zur Zeit und am Ort ihrer Verwendung in einem kontinuierlichen Vorgang mechanisch zerkleinert und die Substanz im Durchflußverfahren in Wasser gelöst, um das flüssige Grundkonzentrat zu erzeugen. Eine diesen Zwecken entsprechende Anordnung ist in Fig.8 schematisch dargestellt. Das stabförmige Stück festen Konzentrats 120 wird mittels einer geriffelten Walze 121 gemahlen, und das gebildete Pulver fällt in die Kammer 103. Die übrigen in Fig.8 dargestellten Elemente entsprechen in ihrer Funktion denen von Fig.7.

Der Zusatz des für die Bildung der Lösung notwendigen Wassers wird vorzugsweise auf der Grundlage einer Messung der Leitfähigkeit der Lösung geregelt, im Sinne eines automatischen Leitfähigkeits-Regelkreises. Ähnlich wie bei der Anwendung des vorgefertigten flüssigen Grundkonzentrats kann mit entsprechender Wahl des Leitfähigkeitsniveaus ein flüssiges Grundkonzentrat gebildet und den Dialysegeräten zugeführt werden, das dann durch die Zufuhr von Wasser auf die endgültige Konzentration verdünnt wird. Mit einer anderen Einstellung des Leitfähigkeitsniveaus, entsprechend der Zufuhr eines höheren Wasseranteils beim Lösungsvorgang, ist es jedoch möglich, direkt eine Grund-Dialysierflüssigkeit zu erzeugen, der nur das Individualkonzentrat zugesetzt werden muß, um die gebrauchsfertige Dialysierflüssigkeit zu erhalten.

Die in Fig.7 und Fig.8 schematisch dargestellten Einrichtungen sind besonders als Bestandteil der Zentralversorgungseinheit 2 in Fig.6 geeignet. Sie können aber auch in Verbindung mit einem einzelnen Dialysegerät verwendet werden und dort die konventionelle Proportionierungseinrichtung ersetzen.

## Patentansprüche

1. Verfahren zur Bereitung von bicarbonathaltigen Dialysierflüssigkeiten für die Hämodialyse in Form wässriger Lösungen, deren Zusammensetzung gemäß den Bereichsangaben der folgenden Tabelle
| Komponente | Konzentration (mmol/Liter) |
|---|---|
| Na+ | 135 - 145 |
| K+ | 0 - 5 |
| Ca++ | 0,3 - 2,5 |
| Mg++ | 0 - 1,2 |
| Cl- | 90 - 125 |
| Acetat- | 0 - 5 |
| Glucose | 0 - 15 |
variabel und individuell an unterschiedliche physiologische Anforderungen der einzelnen Patienten anpassbar ist, unter Verwendung von zwei Konzentraten, von denen das erste die Gesamtmenge des für die Bereitung der Dialysierflüssigkeit notwendigen Natriumbicarbonats und das zweite die für die Bereitung der Dialysierflüssigkeit notwendigen Gesamtmengen von Calcium- und Magnesiumsalzen sowie eine zur Einstellung des pH-Werts notwendige physiologisch verträgliche Säure enthält, **dadurch gekennzeichnet dass**
(a) als erstes Konzentrat ein für den gesamten Variationsbereich einheitliches, hauptsächlich Natriumchlorid und Natriumbicarbonat enthaltendes Konzentrat (Grundkonzentrat) mit einem molaren Verhältnis Bicarbonat/Natrium von vorzugsweise etwa 0,27 und maximal 0,3 verwendet wird und das zweite Konzentrat (Individualkonzentrat) die übrigen Lösungsbestandteile in individuell nach physiologischen Erfordernissen ausgewählter Zusammensetzung liefert,
(b) das Grundkonzentrat Natriumchlorid in einer Konzentration enthalt, die mindestens 80 Prozent, typischerweise jedoch weniger als 95 Prozent der für die Dialysierflüssigkeit vorgesehenen Gesamtkonzentration von Natriumchlorid entspricht, so dass das Grundkonzentrat, bezogen auf die gebrauchsfertige Dialysierflüssigkeit, eine als Standardwert vorgegebene Konzentration der Natriumionen liefert, die an der unteren Grenze des in Betracht kommenden physiologischen Variationsbereiches liegt, wobei der genannte Standardwert im Bereich zwischen 120 und 135 mmol/Liter gewählt ist und vorzugsweise etwa 130 mmol/Liter beträgt,
(c) das Individualkonzentrat ein 120- bis 250-faches, vorzugsweise 170-faches flüssiges Konzentrat ist, das einen Anteil von Natriumionen liefert, der der Differenz zwischen dem genannten Standardwert und dem vorgesehenen individuell angepassten Wert der Natriumkonzentration in der gebrauchsfertigen Dialysierflüssigkeit entspricht, sowie Kalium-, Calcium- und Magnesiumionen in individuell ausgewählten Konzentrationen entsprechend den Angaben der folgenden Tabelle (in mmol/Liter, bezogen auf die gebrauchsfertige Dialysierflüssigkeit):
| | mittlerer Wert | bevorzugter Bereich | maximaler Bereich |
|---|---|---|---|
| Kaliumionen | 2 | 1...4 | (0...5) |
| Calciumionen | 1,6 | 0,75...2 | (0,3...2,5) |
| Magnesiumionen | 0,6 | 0,4...1 | (0...1,2) |

2. Verfahren nach Anspruch 1, **gekennzeichnet durch** die Verwendung eines Individualkonzentrats, das Glucose gemäß folgenden Angaben (in mmol/Liter, bezogen auf die gebrauchsfertige Dialysierflüssigkeit) liefert:
| | mittlerer Wert | bevorzugter Bereich | maximaler Bereich |
|---|---|---|---|
| Glucose | 5 | 0...10 | (0...17) |

3. Verfahren nach Anspruch 1 oder 2, **gekennzeichnet durch** die Verwendung eines Individualkonzentrats, das Salzsäure in einer Konzentration bis zu 4 mmol/Liter, bezogen auf die gebrauchsfertige Dialysierflüssigkeit, enthält.

4. Verfahren nach einem der vorangehenden Ansprüche, **gekennzeichnet durch** die Verwendung eines Grundkonzentrats, das Acetationen bis zu einem molaren Verhältnis Acetat/Natrium von 0,03 enthält und ein flüssiges 15- bis 18-faches Konzentrat, bezogen auf das Volumen der gebrauchsfertigen Dialysierflüssigkeit, darstellt.

5. Verfahren nach einem der vorangehenden Ansprüche, **dadurch gekennzeichnet, dass** das Grundkonzentrat aus zwei einzelnen flüssigen Komponenten besteht.

6. Verfahren nach einem der Ansprüche 1 bis 3, **dadurch gekennzeichnet, dass** das Grundkonzentrat als Trockensubstanz eingesetzt wird und daraus am Ort seiner Anwendung durch Lösen in Wasser in einer Zwischenstufe des Verfahrens ein mindestens 5-faches flüssiges Grundkonzentrat, bezogen auf das Gesamtvolumen der gebrauchsfertigen Dialysierflüssigkeit, gebildet wird.

7. Verfahren nach einem der Ansprüche 1 bis 3, **dadurch gekennzeichnet, dass** das Grundkonzentrat als Trockensubstanz eingesetzt wird und die Trockensubstanz in Wasser gelöst und in Verbindung mit dem Lösungsvorgang in einem der Endkonzentration der Dialysierflüssigkeit entsprechenden Verhältnis mit Wasser verdünnt wird.

8. Verfahren nach Anspruch 6 oder 7, **dadurch gekennzeichnet, dass** die Trockensubstanz des Grundkonzentrats in Form vorgefertigter Stücke von vorgegebener, annähernd gleichmäßige: Form und Größe zur Anwendung gelangt, die in einem kontinuierlichen Vorgang zerkleinert werden und die zerkleinerte Substanz im Durchfluss in dem Wasser gelöst wird.

9. Verfahren nach einem der vorangehenden Ansprüche, **dadurch gekennzeichnet, dass** das Grundkonzentrat bzw. die daraus gebildete Lösung mehreren Hämodialysegeräten gemeinsam über ein Leitungssystem zugeführt wird.

10. Verfahren nach einem der vorangehenden Ansprüche, **dadurch gekennzeichnet, dass** das Individualkonzentrat kontinuierlich im Durchfluss einer Grund-Dialysierflüssigkeit zugemischt wird, die durch Lösen/Verdünnen des Grundkonzentrats gebildet wurde.

11. Verfahren nach einem der Ansprüche 1 bis 9, **dadurch gekennzeichnet, dass** durch Mischen von Wasser und Individualkonzentrat im Durchfluss in einem vorgegebenen Verhältnis eine Lösung gebildet und dieser das Grundkonzentrat im Durchfluss in einem zweiten vorgegebenen Verhältnis zudosiert wird.

12. Verfahren nach Anspruch 11, **dadurch gekennzeichnet, dass** das Individualkonzentrat in die Wasserversorgungsleitung eines Hämodialysegerätes dosiert und dem Wasser zugemischt wird.

13. Verfahren nach einem der vorangehenden Ansprüche, **dadurch gekennzeichnet, dass** mehr als ein Individualkonzentrat parallel oder in zeitlicher Folge zugeführt werden.

## Claims

1. A process for the preparation of bicarbonate containing dialysis fluids for hemodialysis in the form of aquous solutions, the composition thereof being variably and individually adaptable to differing physiological requirements of individual patients in accordance with the ranges of the following table
| component | concentration (mmoles/litre) |
|---|---|
| Na⁺ | 135 - 145 |
| K⁺ | 0-5 |
| Ca⁺⁺ | 0.3 - 2.5 |
| Mg⁺⁺ | 0 - 1.2 |
| Cl⁻ | 90 - 125 |
| acetate⁻ | 0 - 5 |
| glucose | 0 - 15 |
by the use of two concentrates, the first concentrate containing the total amount of sodiumbicarbonate required for the preparation of the dialysis fluid, and the second concentrate containing the total amounts of calcium and magnesium salts required for the preparation of the dialysis fluid as well as a physiologically acceptable acid required for pH-adjustment, charactrized in that
(a) as the first concentrate, a concentrate (basis concentrate) is used that is uniform all over its range and contains mainly sodiumchloride and sodiumbicarbonate at a molar ratio bicarbonate/sodium of, preferably, about 0.27, and 0.3 at the most, and the second concentrate (individual concentrate) provides the residual constituents of the solution at an individual composition selected in accordance with physiological requirements,
(b) the basis concentrate contains sodiumchloride at a concentration that corresponds at least for 80 percent, typically however for less than 95 percent to the total concentration of sodiumchloride provided for the dialysis fluid, so that, related to the ready for use dialysis fluid, the basis concentrate produces a concentration of sodium ions predetermined as the standard and lying at the lower limit of the concerned physiological range, said standard being selected in the range between 120 and 135 mmoles/litre and running preferably to about 130 mmoles/litre,
(c) the individual concentrate is a 120-fold to 150-fold, preferably a 170-fold liquid concentrate providing a share in sodium ions which corresponds to the difference between said standard and the predetermined individually adapted value of the sodium concentration in the ready for use dialysis fluid, as well as potassium, calcium and magnesium ions at individually selected concentrations corresponding to the data in the following table (in mmoles/litre, related to the ready for use dialysis fluid):
| | mean value | preferred range | maximum range |
|---|---|---|---|
| potassium ions | 2 | 1 ... 4 | (0 ... 5) |
| calcium ions | 1.6 | 0.75 ... 2 | (0.3 ... 2.5) |
| magnesium ions | 0.6 | 0.4 ... 1 | (0 ... 1.2) |

2. The process of claim 1, **characterized by** the use of an individual concentrate that provides glucose according to the following data (in mmoles/litre, related to the ready for use dialysis fluid):
| | mean value | preferred range | maximum range |
|---|---|---|---|
| glucose | 5 | 0 ... 10 | (0 ... 17) |

3. The process of claim 1 or 2, **characterized by** the use of an individual concentrate that contains hydrochloric acid with a concentration of up to 4 mmoles/litre, related to the ready for use dialysis fluid.

4. The process of one of the precedent claims, **characterized by** the use of a basis concentrate which contains acetate ions up to a molar ratio acetate/sodium of 0.03 and which constitutes a liquid 15-fold to 18-fold concentrate, related to the volume of the ready for use dialysis fluid.

5. The process of one of the precedent claims, **characterized in that** the basis concentrate consists of two individual liquid components.

6. The process of one of claims 1 to 3, **characterized in that** the basis concentrate is used as the dry substance and **in that**, at the site of its application, related to the total volume of the ready for use dialysis fluid, an at least 5-fold liquid basis concentrate is formed by dissolution in water in an intermediate step.

7. The process of one of claims 1 to 3, **characterized in that** the basis concentrate is used as the dry substance and **in that** the dry substance is dissolved in water and, in conjunction with the dissolution process, is diluted with water at a ratio corresponding to the final concentration of the dialysis fluid.

8. The process of claim 6 or 7, **characterized in that** the dry substance of the basis concentrate is used in form of prefabricated pieces having a predetermined approximately uniform shape and size and being comminuted in a continuous process, the comminuted substance being dissolved in through-passing water.

9. The process of one of the precedent claims, **characterized in that** the basis concentrate or the solution formed therefrom is introduced jointly into several hemodialysers through a conduit system.

10. The process of one of the precedent claims, **characterized in that** the individual concentrate is admixed continuously in the passage of a basic dialysis fluid formed by dissolution/dilution of the basis concentrate.

11. The process of one of claims 1 to 9, **characterized in that**, while circulating, a solution is formed by mixing water and the individual concentrate at a predetermined ratio and, while circulating, the basis concentrate is added thereto by metering at a second predetermined ratio.

12. The process of claim 11, **characterized in that** the individual concentrate is dosed while it is fed into the water supply conduit of a hemodialyser and is admixed to the water.

13. The process of one of the precedent claims, **characterized in that** more than one individual concentrate are supplied simultaneously or in turn.

## Revendications

1. Procédé pour la préparation de liquides de dialyse contenant du bicarbonate pour l'hémodialyse en forme de solutions aqueuses dont la composition peut être ajustée variablement et individuellement aux exigences physiologiques différentes des patients individuels en conformité aux indications de portée de la table suivante:
| composant | concentration (mmoles/litre) |
|---|---|
| Na⁺ | 135 - 145 |
| K⁺ | 0-5 |
| Ca⁺⁺ | 0.3 - 2.5 |
| Mg⁺⁺ | 0 - 1.2 |
| Cl⁻ | 90 - 125 |
| acetate⁻ | 0 - 5 |
| glucose | 0 - 15 |
en utilisant deux concentrés dont le premier contient la quantité totale du bicarbonate de sodium requise pour la préparation du liquide de dialyse et le second contient les quantités totales des sels de calcium et de magnésium requises pour la préparation du liquide de dialyse ainssi qu'un acide physiologiquement inoffensif requis pour l'ajustement du pH,
**caractérisé en ce que**
(a) en tant que premier concentré, on utilise un concentré (concentré de base) qui est unique pour le domaine total et contient principalement le chlorure de sodium et le bicarbonate de sodium, ledit concentré montrant un rapport molaire de bicarbonate/sodium d'environ 0.27, de préférence, et de 0.3, au maximum, et que le second concentré (concentré individuel) fournit le reste des constituants de la solution dans une composition choisie individuellement sur la base des besoins physiologiques,
(b) le concentré de base contient le chlorure de sodium dans une concentration correspondant à 80 pourcents, au minimum, pourtant, caractéristiquement moins de 90 pourcents de la concentration totale pourvue pour le chlorure de sodium dans le liquide de dialyse, au point que le concentré de base fournit une concentration des ions de sodium prédestinée en tant que standard par rapport au liquide de dialyse prêt à l'usage, ladite concentration des ions de sodium en ligne de compte s'élevant à la limite inférieure du domaine de variation physiologique, le standard mentionné étant choisi dans la fourchette entre 120 et 135 mmoles/litre et se montant de préférence à environ 130 mmoles/litre,
(c) le concentré individuel est un concentré liquide 120 fois autant à 250 fois autant, de préférence 170 fois autant, qui fournit une part d' ions de sodium correspondant à la différence entre le standard mentionné et la valeur de la concentration de sodium prévue et ajustée individuellement dans le liquide de dialyse prêt à l'usage, ainssi que des ions de potassium, de calcium et de magnésium de concentrations individuellement choisies correspondant aux données dans la table suivante (en mmoles/litre, par rapport au liquide de dialyse prêt à l'usage):
| | valeur moyenne | fourchette préférée | valeur maximale |
|---|---|---|---|
| ions de potassium | 2 | 1 ... 4 | (0 ... 5) |
| ions de calcium | 1.6 | 0.75 ... 2 | (0.3 ... 2.5) |
| ions de magnésium | 0.6 | 0.4 ... 1 | (0 ... 1.2) |

2. Procédé d'après la revendication 1, **caractérisé par** l'usage d'un concentré individuel, qui fournit le glucose selon les données suivantes (en mmoles/litre, par rapport au liquide de dialyse prêt à l'usage):
| | valeur moyenne | fourchette préférée | valeur maximale |
|---|---|---|---|
| glucose | 5 | 0 ... 10 | (0 ... 17) |

3. Procédé d'après la revendication 1 ou 2, **caractérisé par** l'usage d'un concentré individuel contenant l'acide chlorhydrique d'une concentration allant jusqu'à 4 mmoles/litre par rapport au liquide de dialyse prêt à l'usage.

4. Procédé d'après une des revendications précédentes, **caractérisé par** l'usage d'un concentré de base contenant des ions d'acétate jusqu'à une proportion molaire d' acétate/sodium de 0.03 et représentant un concentré liquide 15 fois autant à 18 fois autant, par rapport au volume du liquide de dialyse prêt à l'usage.

5. Procédé d'après une des revendications précédentes, **caractérisé en ce que** le concentré de base est fait de deux composants liquides individuels.

6. Procédé d'après une des revendications 1 à 3, **caractérisé en ce que** le concentré de base est utilisé en tant que matière solide et que, dans une étape intermédiaire du procédé, on en produit à l'endroit de son usage par dissolution dans l'eau un concentré 5 fois autant, au minimum, par rapport au volume total du liquide de dialyse prêt à l'usage.

7. Procédé d'après une des revendications 1 à 3, **caractérisé en ce que** le concentré de base est utilisé en tant que matière solide et que ladite matière solide est dissoute dans l'eau et, conjointement avec la dissolution, est diluée avec l'eau dans une rapport correspondant à la concentration finale du liquide de dialyse.

8. Procédé d'après la revendication 6 ou 7, **caractérisé en ce que** la matière solide du concentré de base est utilisée sous forme de pièces préfabriquées de'une forme et taille presque régulière, lesdites pièces étant concassées dans un processus continu et la matière concassée étant dissoute dans l'eau en passant.

9. Procédé d'après une des revendications précédentes, **caractérisé en ce que** plusieurs dispositifs d'hémodialyse sont alimentés conjointement en le concentré de base ou, selon le cas, en la solution en produite au moyen d'une canalisation.

10. Procédé d'après une des revendications précédentes, **caractérisé en ce qu'**en passant, un liquide de dialyse de base est additionné continuellement du concentré individuel, ledit liquide de dialyse de base étant produit par la dissolution/dilution du concentré de base.

11. Procédé d'après une des revendications 1 à 9, **caractérisé en ce qu'**en mélanger de l'eau avec le concentré individuel dans un rapport prédéterminé, une solution est produite en passant à laquelle le concentré de base est dosé en passant dans un second rapport prédéterminé.

12. Procédé d'après la revendication 11, **caractérisé en ce que** l'eau est additionné du concentré individuel dosé dans la conduite d'alimentation en eau d'un hémodialyseur.

13. Procédé d'après une des revendications précédentes, **caractérisé en ce que** l'on alimente simultanément ou successivement en plus d'un concentré individuel.
